# EUROPEAN PATENT SPECIFICATION

(11) **EP 1 277 720 B1**
(45) Date of publication and mention of the grant of the patent: **11.01.2006**
(21) Application number: 01309599.7
(22) Date of filing: 14.11.2001
(51) Int. Cl.: C07C 21/18, C07C 19/08, C07C 19/10, C07C 17/00, C07C 17/20

(54) **Fluorination of 1,1,1,3,3-pentachloropropane**
Verfahren zur Fluorierung von 1,1,1,3,3-Pentachloropropan
Procédé pour la fluorination du 1,1,1,3,3-pentachloropropane

(30) Priority: 20.07.2001 US 910375
(43) Date of publication of application: 22.01.2003
(73) Proprietor: Arkema Inc., Philadelphia, PA 19103-3222 (US)
(72) Inventor: Bolmer, Michael S., Collegeville, Pennsylvania 19426 (US); Chen, Bin, Wayne, Pennsylvania 19087 (US)
(74) Representative: Stoner, Gerard Patrick

(56) References cited:
- US-A- 5 616 819
- DATABASE WPI Section Ch, Week 199937 Derwent Publications Ltd., London, GB; Class A60, AN 1999-439409 XP002226126 & JP 11 180907 A (DAIKIN KOGYO KK), 6 July 1999 (1999-07-06)

## Description

This invention relates to the uncatalyzed fluorination of 1,1,1,3,3-pentachloropropane ("240fa") in the presence of a solvent to produce fluorinated products selected from one or more of CClₐF₃₋ₐCH=CHCl and CCl_{b}F_{3-b}CH₂CHCl_{c}F_{2-c} where a, b and c are each equal to 0, 1 or 2. The fluorinated product 245fa (where b and c equal 0) is useful as a foam blowing agent and refrigerant. The other fluorinated products are intermediates for 245fa.

Previous attempts to fluorinate 240fa without the use of catalysts, as disclosed in Japanese Patent Applications JP-A-10/101593, JP-A-11/180908 and JP-A-11/180907, have resulted in a low level of fluorination, while catalyzed fluorination of 240fa, such as with antimony pentachloride, increases corrosivity of the reactants and selectivity to undesired oligomer formation.
US-A-5616819 discloses a two-step process in which in a first uncatalysed step a chloropropene is fluorinated to a chlorofluoropropene using HF, optionally in a solvent such as sulfolane (tetramethylene sulfone). In a second, catalysed step this chlorofluoropropene is fluorinated using HF to the target fluoropropane such as 245fa.
It has now been found that use of tetramethylene sulfone as a solvent in the uncatalysed fluorination of 240fa reduces the formation of heavy by-products and increases the reaction rate and the extent of fluorination. The extent of fluorination (E) is determined by dividing the number of fluorines added to a molecule by the number of chlorine and double bonds on the reactant.
Thus, the invention provides an uncatalysed process for the fluorination of 1,1,1,3,3-pentachloropropane, which comprises:
(a) contacting 1,1,1,3,3-pentachloropropane with hydrogen fluoride in the presence of tetramethylene sulfone, at a molar ratio of hydrogen fluoride to 1,1,1,3,3-pentachloropropane of from 10:1 to 200:1 and at temperature from 75°C to 125°C, to produce fluorinated products being one or more of CClₐF₃₋ₐCH=CHCl and CCl_{b}F_{3-b}CH₂CHCl_{c}F_{2-c}, where a, b and c are independently selected from 0, 1 and 2,and
(b) separating the fluorinated products from the reaction mixture resulting from (a).

The process of this invention can be conducted as a batch or semi-continuous process. The HF:240fa molar ratio is from 10 to 200, preferably from 20 to 100. The tetramethylene sulfone:240fa molar ratio is typically from 0.5 to 20, preferably from 1 to 5. The temperature is from 75 to 125°C. The gauge pressure is typically from about 0 to 3.45MPa (0 to 500 psig), preferably from 0.83 to 1.72MPa (120 to 250 psig). Residence time is typically from about 5 minutes to 8 hours, preferably from about 0.5 to 3 hours. The by-product HCl can be removed from the resultant reaction mixture by methods known in the art such as by absorption (in water or caustic) or by distillation. The HCl can also be continuously removed from the reactor during the reaction by distillation. The practice of the invention is illustrated in more detail in the following non-limiting example.

14.4 g of 240fa, 116 g of HF and 8.4 g of tetramethylene sulfone were placed in an autoclave (HF/240fa = 90:1). The contents were heated to 100°C for 3 hours. The pressure reached 1.93 MPa (280 psig). Conversion was 100%. Selectivity was 7.8% for 241fa (b and c = 2), 0.7% for 242fa (b = 1 and c = 2), 0.4% for 1232zd (a = 1), 0.2% for 243fa (b = 0 and c = 2), 69.5% for 1233zd (a = 0), 1.9% for 244fa (b = 0 and c = 1), 18.1% for 245fa (b and c = 0), and 1.4% for others. E = 0.63. By comparison, when the solvent was omitted, E was only 0.37, conversion was 83%, and selectivity was only 2.5% for fluorinated products other than 241fa and 1233zd.

## Claims

1. An uncatalysed process for the fluorination of 1,1,1,3,3-pentachloropropane, which comprises:
(a) contacting 1,1,1,3,3-pentachloropropane with hydrogen fluoride in the presence of tetramethylene sulfone, at a molar ratio of hydrogen fluoride to 1,1,1,3,3-pentachloropropane of from 10:1 to 200:1 and at temperature from 75°C to 125°C, to produce fluorinated products being one or more of CClₐF₃₋ₐCH=CHCl and CCl_{b}F_{3-b}CH₂CHCl_{c}F_{2-c}, where a, b and c are independently selected from 0, 1 and 2, and
(b) separating the fluorinated products from the reaction mixture resulting from (a).

2. A process according to claim 1 in which said molar ratio of hydrogen fluoride to 1,1,1,3,3-pentachloropropane is from 20:1 to 100:1.

3. A process according to claim 1 or 2 in which the molar ratio of tetramethylene sulfone to 1,1,1,3,3-pentachloropropane is from 0.5:1 to 20:1.

4. A process according to claim 3 in which the molar ratio of tetramethylene sulfone to 1,1,1,3,3-pentachloropropane is from 1:1 to 5:1.

5. A process according to any one of the preceding claims producing CF₃CH₂CHF₂.

## Patentansprüche

1. Unkatalysiertes Verfahren für die Fluorierung von 1,1,1,3,3-Pentachlorpropan, umfassend:
(a) Inkontaktbringen von 1,1,1,3,3-Pentachlorpropan mit Fluorwasserstoff in Gegenwart von Tetramethylensulfon bei einem Molverhältnis von Fluorwasserstoff zu 1,1,1,3,3-Pentachlorpropan von 10:1 bis 200:1 und bei einer Temperatur von 75°C bis 125°C unter Herstellung von fluorierten Produkten, die eins oder mehrere von CClₐF₃₋ₐCH=CHCl und CCl_{b}F_{3-b}CH₂CHCl_{c}F_{2-c}, worin a, b und c unabhängig aus 0, 1 und 2 ausgewählt sind, sind und
(b) Abtrennen der fluorierten Produkte aus dem Reaktionsgemisch, das aus (a) resultiert.

2. Verfahren nach Anspruch 1, wobei das Molverhältnis von Fluorwasserstoff zu 1,1,1,3,3-Pentachlorpropan 20:1 bis 100:1 ist.

3. Verfahren nach Anspruch 1 oder 2, wobei das Molverhältnis von Tetramethylensulfon zu 1,1,1,3,3-Pentachlorpropan 0,5:1 bis 20:1 ist.

4. Verfahren nach Anspruch 3, wobei das Molverhältnis von Tetramethylensulfon zu 1,1,1,3,3-Pentachlorpropan 1:1 bis 5:1 ist.

5. Verfahren nach einem der vorangehenden Ansprüche, das CF₃CH₂CHF₂ produziert.

## Revendications

1. Procédé non catalysé pour la fluoration du 1,1,1,3,3-pentachloropropane, qui comprend :
(a) la mise en contact de 1,1,1,3,3-pentachloropropane avec du fluorure d'hydrogène en présence de tétraméthylènesulfone, en un rapport molaire du fluorure d'hydrogène au 1,1,1,3,3-pentachloropropane de 10:1 à 200:1 et à une température de 75°C à 125°C, pour produire des produits fluorés consistant en un ou plusieurs des composés de formules CClₐF₃₋ₐCH=CHCl et CCl_{b}F_{3-b}CH₂CHCl_{c}F_{2-c}, dans lesquelles a, b et c sont choisis indépendamment entre 0, 1 et 2, et
(b) la séparation des produits fluorés du mélange réactionnel résultant de (a).

2. Procédé suivant la revendication 1, dans lequel ledit rapport molaire du fluorure d'hydrogène au 1,1,1,3,3-pentachloropropane est compris dans l'intervalle de 20:1 à 100:1.

3. Procédé suivant la revendication 1 ou 2, dans lequel ledit rapport molaire de la tétraméthylènesulfone au 1,1,1,3,3-pentachloropropane est compris dans l'intervalle de 0,5:1 à 20:1.

4. Procédé suivant la revendication 3, dans lequel ledit rapport molaire de la tétraméthylènesulfone au 1,1,1,3,3-pentachloropropane est compris dans l'intervalle de 1:1 à 5:1.

5. Procédé suivant l'une quelconque des revendications précédentes, produisant du CF₃CH₂CHF₂.
